# EUROPEAN PATENT APPLICATION

(11) **EP 3 467 705 A1**
(43) Date of publication of application: **10.04.2019**
(21) Application number: 17802169.7
(22) Date of filing: 24.05.2017
(51) Int. Cl.: G06K 9/00

(54) **PHOTOSENSITIVE IMAGE ELEMENT, IMAGE COLLECTOR, FINGERPRINT COLLECTION DEVICE, AND DISPLAY DEVICE**

(30) Priority: 25.05.2016 CN 201610357410
(71) Applicant: Vkansee (Beijing) Technology Co., Ltd., Beijing 100193 (CN)
(72) Inventor: ZHANG, Mingfang, Beijing 100193 (CN); NIU, Xingyu, Beijing 100193 (CN)
(74) Representative: Patent- und Rechtsanwälte Ullrich & Naumann
(86) International application number: PCT/CN2017/085604
(87) International publication number: WO 2017/202323

(57) **Abstract**

A photosensitive image element, an image collector, a fingerprint collection device, and a display device supporting a collection function. The photosensitive image element comprises a chamber (1) for accommodating an optical-to-electrical conversion unit. A light blocking film (3) in which light-transmission pinholes (2) are formed covers above the chamber (1). A transparent medium layer (4) and a microlens (5) are sequentially disposed above the light blocking film (3). The micro lens (5), the transparent medium layer (4) and the light-transmission pinholes (2) enable the photosensitive image element to have an object field (6) for limiting a field angle. An image point or an image speckle of an object point within the object field (6) falls within a corresponding light-transmission pinhole (2) in the light blocking film (3), and an image point or an image speckle of the object point outside the object field (6) falls outside a corresponding light-transmission pinhole (2). The pinholes and the microlens on the light blocking film constrain the incident optical path, so that the photosensitive image element has a narrow visual field, and the image collector based on an photosensitive image element having a narrow visual field does not need to depend on a lens system or a pinhole imaging system to constrain the incident optical path, and accordingly while the image collector can obtain a clear image, a thinner image collector becomes possible, which helps the image collector integrate with another device.

## Description

The present application claims priority of Chinese Patent Application No. 201610357410.0, filed with the Chinese Patent Office on May 25, 2016 and entitled "IMAGING PIXEL, IMAGE ACQUISITION DEVICE, FINGERPRINT ACQUISITION APPARATUS, AND DISPLAY APPARATUS", which is incorporated herein by reference in its entirety.

### TECHNICAL FIELD

The present application relates to the field of image acquisition technologies, and specifically to an imaging pixel with a narrow angle of view, an image acquisition device using the imaging pixel, and an optical fingerprint acquisition apparatus.

### BACKGROUND OF THE INVENTION

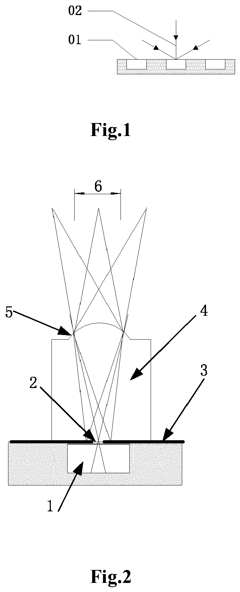

In an image acquisition device, a minimum unit for receiving an optical signal is an imaging pixel, and the image acquisition device is usually provided with several imaging pixels. Imaging pixels used in commercially available image acquisition devices all have a wide angle of view. An angle of view of an imaging pixel refers to a maximum angle formed by incident light rays from different directions to which the imaging pixel is able to respond. Fig. 1 is a schematic diagram illustrating an angle of view of an imaging pixel in an existing image acquisition device. As shown in Fig. 1, an angle of view of an imaging pixel 01 in the existing image acquisition device is nearly 180 degrees, i.e., a maximum angle between incident light rays 02 is nearly 180 degrees.

When an image is acquired directly by using the existing image acquisition device, the acquired image is usually not clear due to a wide angle of view. In this case, a lens system or a pinhole imaging system needs to be used to confine an incident light path, so that each imaging pixel responds only to light rays at a certain angle in an object-side field of view, so as to acquire a clear image.

Because a structure such as a lens system is used, an existing image acquisition apparatus is usually very thick. However, as electronic devices are trending to be required to be ultra-thin, a thick image acquisition apparatus cannot meet the requirement of manufacturers. Therefore, there is a need of providing an image acquisition apparatus with a narrow field of view that can both meet the production requirement of the manufacturers for being ultra-thin and acquire a clear image.

### SUMMARY OF THE INVENTION

The invention objective of the present application is to provide an image acquisition device that has a narrow field of view, can meet a production requirement of manufacturers for being ultra-thin, and can acquire a clear image, an image acquisition apparatus using the image acquisition device, a fingerprint acquisition apparatus, a display screen with a fingerprint acquisition function, etc.

According to one aspect of the present application, an imaging pixel is provided, including a cavity in which an optical-to-electrical conversion unit is accommodated. A light blocking film with a light transmission pinhole covers over the cavity, and a transparent medium layer and a microlens are sequentially disposed on the light blocking film from bottom to top.

An image plane of an object plane in a set area above the imaging pixel falls on a plane where the light blocking film locates. The microlens, the transparent medium layer, and the light transmission pinhole are provided so that the imaging pixel has an object-side field of view with a confined angle of view on the object plane in the set area. An image point or an image spot of an object point within the object-side field of view falls within the light transmission pinhole of the light blocking film; and an image point or an image spot of an object point outside the object-side field of view falls outside the light transmission pinhole.

Preferably, the microlens is a curved convex lens.

As one of preferred solutions, a line (referred to as an optical axis below) connecting centers of the light transmission pinhole and the microlens is perpendicular to an upper surface of the light blocking film (this case is referred to as an orthogonal object-side field of view).

As another preferred solution, the optical axis is not perpendicular to the upper surface of the light blocking film (this case is referred to as an oblique object-side field of view).

Preferably, the angle of view corresponding to the object-side field of view is less than 10 degrees.

The light blocking film is a film made of an opaque material, and the light blocking film has a thickness less than 100 um.

According to another aspect of the present application, an image acquisition device is further provided, including several imaging pixels described above. The several imaging pixels are arranged in an array, and a low-refractive-index layer and a transparent cover plate are sequentially disposed on the several imaging pixels from bottom to top.

An image point of an object point within an object-side field of view on an upper surface of the transparent cover plate corresponding to each of the imaging pixels falls within a light transmission pinhole of the light blocking film; and an image point of an object point outside the object-side field of view falls outside the light transmission pinhole.

Preferably, between adjacent imaging pixels, a light blocking wall is disposed in a transparent medium layer between a microlens and the light blocking film, to prevent signal crosstalk between the adjacent imaging pixels.

An angle of view of the object-side field of view of each imaging pixel is *α*=2arctan(*r_{object}*//*h*), where *r_{object}* is a radius of the object-side field of view on the upper surface of the transparent cover plate corresponding to the imaging pixel, and h is the height from the upper surface of the transparent cover plate to a center of the light transmission pinhole.

As one of preferred solutions, all of the imaging pixels included in the image acquisition device have an orthogonal object-side field of view, and are located on a same planar substrate.

As one of preferred solutions, all the imaging pixels included in the image acquisition device have a same oblique object-side field of view and have parallel optical axes, and are located on a same planar substrate.

As one of preferred solutions, the imaging pixels are located on a curved substrate convex or concave to the transparent cover plate.

As one of preferred solutions, the imaging pixels included in the image acquisition device are regularly arranged from inside toward outside with respect to one or some of the imaging pixels as a center, the imaging pixel as the center has an orthogonal object-side field of view, and other imaging pixels each have an oblique object-side field of view; and from the center toward outside, optical axes of the respective imaging pixels gradually oblique outwardly, and angles between the optical axes and an upper surface of the light blocking film gradually decrease.

According to still another aspect of the present application, a fingerprint acquisition apparatus is further provided, including the image acquisition device described above.

According to yet another aspect of the present application, a display apparatus supporting a fingerprint acquisition function is provided, including several display pixels. The imaging pixel described above is disposed between the display pixels.

It can be learned from the foregoing technical solutions that, the imaging pixel in the present application incorporates the transparent medium layer and the microlens, and the imaging pixel can confine an incident light path by using the pinhole in the light blocking film and the microlens, so that the imaging pixel has a narrow field of view with a smaller angle of view. Therefore, an image acquisition device using the imaging pixel can confine an incident light path without the help of a lens system or a pinhole imaging system, so that the image acquisition device can become thinner while acquiring a clear image.

### BRIEF DESCRIPTION OF THE DRAWINGS

To describe the technical solutions in the embodiments of the present invention or the prior art more clearly, the accompanying drawings for the embodiments are simply described below. Apparently, the accompanying drawings in the following description show merely some embodiments, and persons or ordinary skill in the art can derive other drawings from the accompanying drawings without creative efforts.
Fig. 1 is a schematic diagram illustrating an angle of view of an imaging pixel in a conventional image acquisition device;
Fig. 2 is a schematic structural diagram illustrating an imaging pixel according to a preferred embodiment;
Fig. 3 is a schematic structural diagram illustrating an imaging pixel according to another preferred embodiment;
Fig. 4 is a schematic structural diagram illustrating an image acquisition device according to a preferred embodiment;
Fig. 5 is a schematic structural diagram illustrating an image acquisition device according to another preferred embodiment;
Fig. 6 is a schematic structural diagram illustrating an image acquisition device according to another preferred embodiment;
Fig. 7 is a diagram illustrating a structural parameter of an image acquisition device according to a specific embodiment;
Fig. 8 is a schematic structural diagram illustrating an image acquisition device according to another preferred embodiment;
Fig. 9 is a schematic structural diagram illustrating an image acquisition device according to another preferred embodiment;
Fig. 10 is a schematic structural diagram illustrating an image acquisition device according to another preferred embodiment;
Fig. 11 is a schematic structural diagram illustrating an image acquisition device according to another preferred embodiment;
Fig. 12 is a schematic structural diagram illustrating an image acquisition device according to another preferred embodiment; and
Fig. 13 is a schematic structural diagram illustrating a display apparatus supporting a fingerprint acquisition function according to a preferred embodiment.

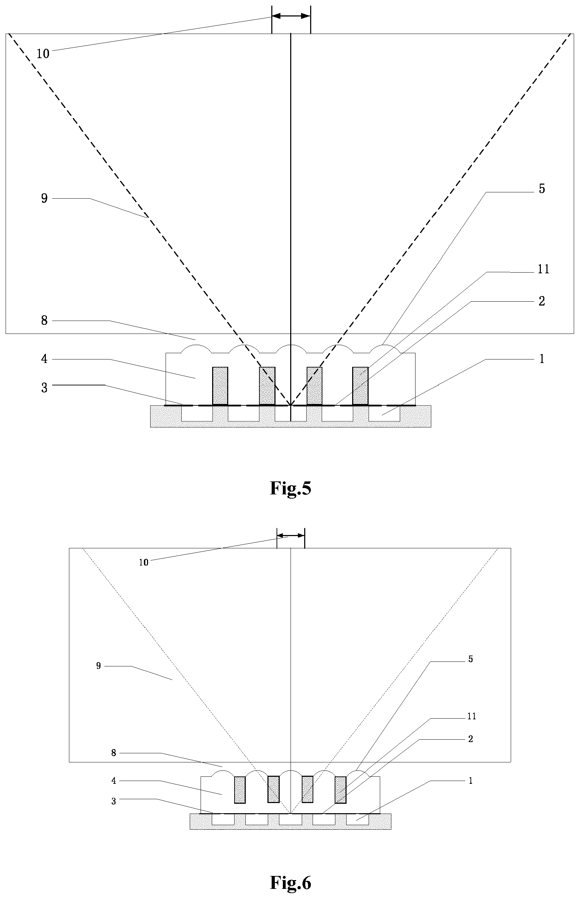

Note: For clarity, in Fig. 5, Fig. 6, Fig. 9, Fig. 10, Fig. 11, and Fig. 12, only an optical axis of each imaging pixel is shown.

### DETAILED DESCRIPTION OF THE EMBODIMENTS

Exemplary embodiments of the present invention will be described below in more detail with reference to the accompanying drawings for the embodiments so that the technical solutions in accordance with the embodiments of the present invention are more clear and complete. Apparently, the described embodiments are merely some rather than all of the embodiments of the present invention. All other embodiments achieved by persons of ordinary skill in the art based on the embodiments of the present application without creative efforts shall fall within the protection scope of the present application.

Fig. 2 is a schematic structural diagram illustrating an imaging pixel according to a preferred embodiment. As shown in Fig. 2, the imaging pixel includes a cavity 1 in which an optical-to-electrical conversion unit is accommodated. A light blocking film 3 with a light transmission pinhole 2 covers over the cavity 1. A transparent medium layer 4 and a microlens 5 are sequentially disposed on the light blocking film 3 from bottom to top. Preferably, the microlens 5 in the present application is a curved convex lens. A line (i.e., an optical axis) connecting centers of the light transmission pinhole 2 and the microlens 5 is perpendicular to an upper surface of the light blocking film 3, so that a center of an object-side field of view is on a central normal of the imaging pixel (this case indicates an orthogonal object-side field of view). Preferably, the light blocking film3 is a film made of an opaque material, and the light blocking film 3 has a thickness of less than 100 um.

An image plane of an object plane in a set area above the imaging pixel falls on a plane where the light blocking film locates. An angle of view of the imaging pixel in the present application may be confined by using a structure of the light transmission pinhole 2, the microlens 5, and the transparent medium layer 4. The angle of view corresponds to an object-side field of view 6. As a preferred embodiment in various embodiments, the angle of view of the imaging pixel in this embodiment is less than 10 degrees. An image point or an image spot of an object point within the object-side field of view 6 confining the angle of view falls within the light transmission pinhole 2 of the light blocking film 3; and an image point or an image spot of an object point outside the object-side field of view 6 falls outside the light transmission pinhole 2. The object-side field of view of the imaging pixel in the present application may be referred to as a narrow object-side field of view.

It should be noted that, it is merely an example that the angle of view of the imaging pixel is less than 10 degrees, and persons skilled in the art may achieve a corresponding angle of view by adjusting a focal length of the microlens, a thickness of the transparent medium layer, and a size of the light transmission pinhole depending on a production requirement.

The working principle of the imaging pixel in this embodiment is described in detail below.

In this embodiment, the angle of view of the imaging pixel in the present application is confined by using the structure of the light transmission pinhole, the microlens, and the transparent medium layer, and an image point or an image spot of a corresponding object point within the confined object-side field of view mainly falls within the light transmission pinhole 2 of the light blocking film 3, so that light emitted by the object point within the object-side field of view travels into the cavity 1 through the pinhole. Correspondingly, the optical-to-electrical conversion unit can receive light of a higher intensity, so that an image of an object within the object-side field of view can be formed clearly. Because an image point or an image spot of an object point outside the object-side field of view mainly falls outside the light transmission pinhole 2, the optical-to-electrical conversion unit cannot sense light emitted by the object point outside the object-side field of view or can only sense weak light, and an image of an object outside the object-side field of view cannot be acquired or an acquired part does not affect imaging, on the optical-to-electrical conversion unit, by light from within the object-side field of view.

In comparison with an imaging pixel used in a commercially available image acquisition device, the imaging pixel in this embodiment incorporates the thick transparent medium layer 4 and the microlens 5, and the imaging pixel itself can confine an incident light path by using the pinhole in the light blocking film 3 and the microlens 5, so that the imaging pixel has a narrow field of view with a smaller angle of view. Therefore, it is possible that an image acquisition device using the imaging pixel can confine an incident light path, without the help of a lens system or a pinhole imaging system, and can acquire a clear image. Although there is a commercially available image sensor which is also provided with a microlens and so on, its purpose is to increase the amount of incident light during oblique incidence, which is essentially different from the objective and implementation method of the microlens in the present application which enables an imaging pixel to have a narrow field of view.

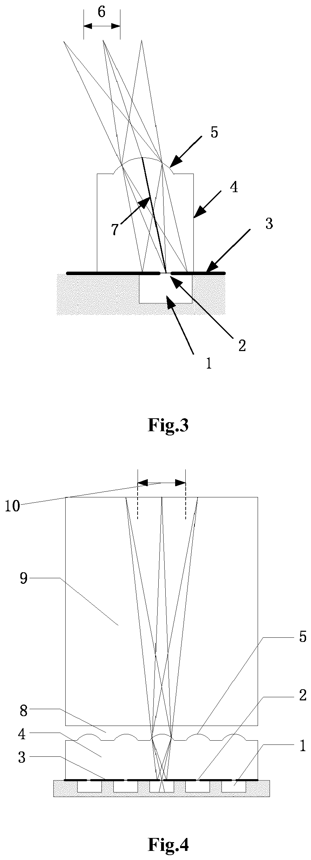

Fig. 3 is a schematic structural diagram illustrating an imaging pixel according to another preferred embodiment. A structure of the imaging pixel shown in Fig. 3 is similar to the structure of the imaging pixel shown in Fig. 2, except that an optical axis of the imaging pixel shown in Fig. 3 is not perpendicular to the upper surface of the light blocking film 3, i.e., the optical axis of the imaging pixel shown in Fig. 3 is set oblique relative to the upper surface of the light blocking film 3, so that a center of an object field of view is not on a central normal of the imaging pixel (this case is an oblique object-side field of view). Similarly, an image point or an image spot of an object point within the oblique object-side field of view 6 confined by the light transmission pinhole 2, the microlens 5, and the transparent medium layer 4 falls within the light transmission pinhole 2 of the light blocking film 3; and an image point or an image spot of an object point outside the oblique object-side field 6 of view falls outside the light transmission pinhole 2. As a preferred embodiment in the various embodiments, an angle of view corresponding to the oblique object-side field of view 6 in this embodiment is less than 10 degrees. In the present application, the value of an angle between a line connecting centers of the light transmission pinhole 2 and the microlens 5 and the upper surface of the light blocking film 3 is not specifically limited, and persons skilled in the art may set the angle between the line connecting the centers of the light transmission pinhole 2 and the microlens 5 and the upper surface of the light blocking film 3 depending on a production requirement.

The work principle of the imaging pixel shown in Fig. 3 is the same as the imaging pixel shown in Fig. 2, and the description thereof is not repeated herein.

Fig. 4 is a schematic structural diagram illustrating an image acquisition device according to a preferred embodiment. As shown in Fig. 4, the image acquisition device includes several imaging pixels shown in Fig. 2 or Fig. 3. The several imaging pixels are arranged in an array and are integrally formed. A low-refractive-index layer 8 and a transparent cover plate 9 are sequentially disposed above the several imaging pixels from bottom to top.

During image acquisition, an image point of an object point within an object-side field of view 10 on an upper surface of the transparent cover plate corresponding to each imaging pixel falls within a light transmission pinhole 2 of a light blocking film 3. An image point of an object point outside the object-side field of view falls outside the light transmission pinhole 2. An angle of view of the object-side field of view of each imaging pixel is *α*=2arctan(*r_{object}*/*h*), where *r_{object}* is a radius of the object-side field of view on the upper surface of the transparent cover plate corresponding to the imaging pixel, and h is the height from the upper surface of the transparent cover plate to a center of the light transmission pinhole 2.

Fig. 5 is a schematic structural diagram illustrating an image acquisition device according to a preferred embodiment. A structure of the image acquisition device shown in Fig. 5 is similar to the structure of the image acquisition device shown in Fig. 4, except that between adjacent imaging pixels, a light blocking wall 11 is provided in a transparent medium layer 4 between a microlens 5 and a light blocking film 3 close to a cavity 1 in which an optical-to-electrical conversion unit is accommodated, so as to prevent signal crosstalk between the imaging pixels. As shown in Fig. 5, crosstalk light rays (shown by dashed lines in the figure) from left and right sides of an object side are blocked and absorbed by the light blocking wall 11 after travelling through the lens 5.

Fig. 6 is a schematic structural diagram illustrating an image acquisition device according to another preferred embodiment. A structure of the image acquisition device shown in Fig. 6 is similar to the structure of the image acquisition device shown in Fig. 4, except that a light blocking wall 11 is disposed in a transparent medium layer 4 between a microlens 5 and a light blocking film 3 close to the microlens 5, so as to prevent signal crosstalk (shown by dashed lines in the figure) between the imaging pixels.

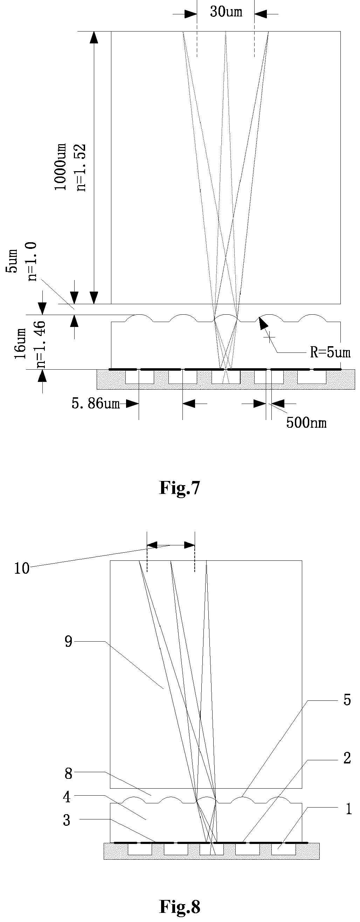

A structure design of an image acquisition device using an imaging pixel having a narrow object-side field of view is further described with reference to a typical embodiment. Fig. 7 shows a diagram illustrating structural parameters of an image acquisition device in a specific embodiment. As shown in Fig. 7, a transparent cover plate has a thickness of 1.0 mm and a refractive index of 1.52. A low-refractive-index layer has a thickness of 5 um (from a lower surface of the transparent cover plate to the top of a microlens layer) and a refractive index of 1.0, and the low-refractive-index layer is air or vacuum. A transparent medium layer has a refractive index of 1.46, and is 16 um thick (including the thickness of the microlens, where 16 um indicates the thickness from the top of the microlens to an upper surface of a light blocking film). The light blocking film is 500 nm thick, and is made of an opaque metal material. A pinhole is a round pinhole with a diameter of 500 nm, and is formed through optical etching. A center of the pinhole is aligned with a center of an imaging pixel. The imaging pixel is square with a side of 5.86 um. The microlens is a spherical convex lens with a radius of 5 um, and a center of the lens is aligned with the center of the photosensitive pixel.

According to calculation and simulation results, an object-side field of view of the imaging pixel with a narrow field of view has a diameter (on an upper surface of the transparent cover plate) of 30 um. Therefore, a radius of the object-side field of view on the upper surface of the transparent cover plate corresponding to the imaging pixel is 15 um. It can be learned from the parameters in the figure that, h=1000+5+16=1021 um, and it can be calculated by the formula *α*=2arctan(*r_{object}*/*h*) that an angle of view of the image acquisition device is 1.68 degrees.

For light within the object-side field of view meeting the angle of view as above, incident light can be confined convergently to an optical-to-electrical conversion unit through limitation by the transparent cover plate, the low-refractive-index layer, the microlens 5, the transparent medium layer 4, and the light transmission pinhole 2, and light beyond the angle of view cannot reach the optical-to-electrical conversion unit, so that a clear image is formed. In addition, because the imaging pixel incorporates the transparent medium layer 4 and the microlens 5, no conventional lens system is needed, so that the image acquisition device can be developed towards being ultra-thin.

Fig. 8 is a schematic structural diagram illustrating an image acquisition device according to another preferred embodiment. A structure of the image acquisition device shown in Fig. 8 is similar to the structure of the image acquisition device shown in Fig. 4, except using an imaging pixel with an oblique object-side field of view. In some cases, for example, during fingerprint image acquisition, due to a gully structure on a fingerprint surface, the image acquisition device using the imaging pixel with the oblique object-side field of view can acquire a clearer image, and experience less interference from stray ambient light.

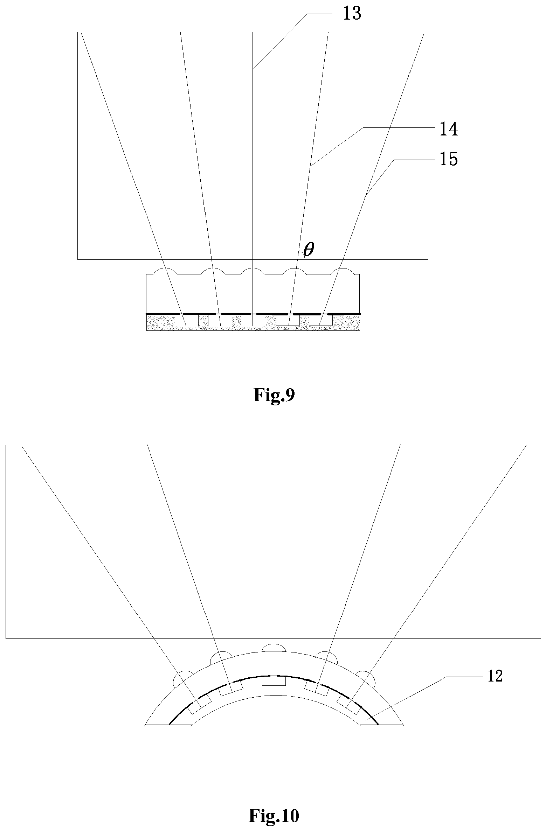

Fig. 9 is a schematic structural diagram illustrating an image acquisition device according to another preferred embodiment. A structure of the image acquisition device shown in Fig. 9 is similar to the structure of the image acquisition device shown in Fig. 4, except using both an imaging pixel with an orthogonal object-side field of view and an imaging pixel with an oblique object-side field of view. On a central area is the imaging pixel with an orthogonal object-side field of view, and outside the central area are all the imaging pixels with an oblique object-side field of view. From the center area toward outside, optical axes (e.g., an optical axis 14 and an optical axis 15 in Fig. 9) of pixels gradually oblique outwardly, angles between the optical axes and an upper surface of a light blocking film gradually decrease, and the optical axes of the pixels are distributed radially. This special imaging pixel arrangement can expand an object-side field of view (referring to an imaging range of the image acquisition device, different from an object-side field of view of a single imaging pixel) of the image acquisition device, to clearly acquire an image within a larger range of space.

Fig. 10 is a schematic structural diagram illustrating an image acquisition device according to another preferred embodiment. A structure of the image acquisition device shown in Fig. 10 is similar to the structure of the image acquisition device shown in Fig. 4, except that an imaging pixel is located on a curved substrate 12 convex to a transparent cover plate, which can also expand an object-side field of view of the image acquisition device, to clearly acquire an image within a larger range of space.

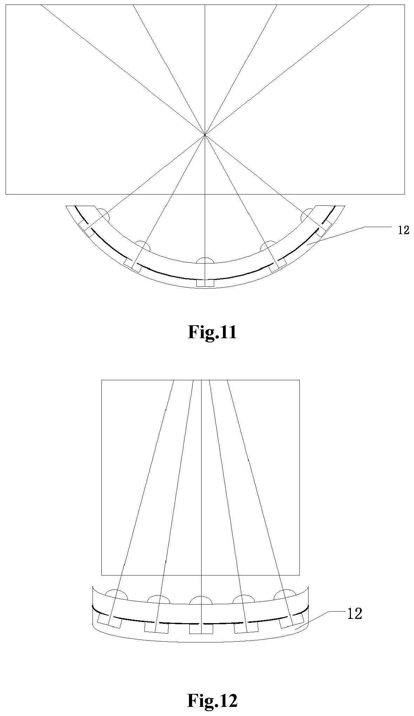

Fig. 11 is a schematic structural diagram illustrating an image acquisition device according to another preferred embodiment. A structure of the image acquisition device shown in Fig. 11 is similar to the structure of the image acquisition device shown in Fig. 4, except that an imaging pixel is located on a curved substrate 12 concave to a transparent cover plate, which can also expand an object-side field of view of the image acquisition device for a farther target, to clearly acquire an image within a larger range of space.

Fig. 12 is a schematic structural diagram illustrating an image acquisition device according to another preferred embodiment. A structure of the image acquisition device shown in Fig. 12 is similar to the structure of the image acquisition device shown in Fig. 4, except that an imaging pixel is located on a curved substrate 12 concave to a transparent cover plate, which can narrow an object-side field of view of the image acquisition device for a closer target, to clearly magnify a small object-side field of view to a larger imaging space, thereby it is possible to achieve magnification of a formed image.

According to another aspect of the present application, a fingerprint acquisition apparatus is further provided, including the image acquisition device in accordance with the various embodiments described above.

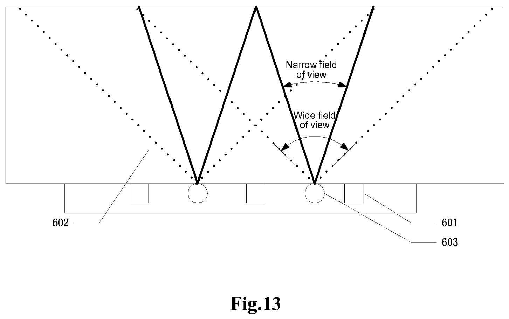

According to still another aspect of the present application, a display apparatus supporting a fingerprint acquisition function is further provided. Fig. 13 is a schematic structural diagram illustrating a display apparatus supporting a fingerprint acquisition function according to a preferred embodiment. As shown in Fig. 13, the display apparatus includes several display pixels 601 and a display panel 602. Imaging pixels 603 with a set angle of view described above are disposed between the display pixels 601. The angle of view of the imaging pixels 603 in this embodiment corresponds to a narrower object-side field of view. The imaging pixels are arranged in an array. The imaging pixels 602 with a narrow object-side field of view can acquire a clear fingerprint image of a fingerprint pressed on the display panel. On the contrary, if the imaging pixels have a wider object-side field of view (also referred to as a wide object-side field of view), object-side fields of view of adjacent imaging pixels will overlap much, resulting in a blurred output image, as shown in Fig. 13.

It can be learned from the foregoing technical solutions that, the imaging pixel in the present application incorporates the transparent medium layer 4 and the microlens 5, and the imaging pixel can confine an incident light path by using the pinhole in the light blocking film 3 and the microlens 5, so that the imaging pixel has a narrow field of view with a smaller angle of view. Therefore, an image acquisition device using the imaging pixel can confine an incident light path without the help of a lens system or a pinhole imaging system, so that the image acquisition device can become thinner while acquiring a clear image.

Persons skilled in the art may readily figure out other implementation solutions of the present application by considering the specification and practicing the invention disclosed herein. The present application is intended to cover any variations, purposes, or adaptations of the present application, which conform to the general principle of the present application and include common general knowledge or a conventional technical means in the art that is disclosed and not disclosed in this disclosure. The specification and the embodiments are merely for an illustrative purpose, and the true scope and spirit of the present application are subject to the following claims.

It should be understood that the present application is not limited to the foregoing described precise methods shown in the drawings, to which various modifications and changes may be made without departing from the scope of the present application. The scope of the present application is subject only to the appended claims.

## Claims

1. An imaging pixel comprising a cavity in which an optical-to-electrical conversion unit is accommodated, wherein the imaging pixel further comprises: a light blocking film with a light transmission pinhole covering over the cavity; and a transparent medium layer and a microlens sequentially disposed on the light blocking film from bottom to top, wherein
an image plane of an object plane in a set area above the imaging pixel falls on a plane where the light blocking film locates; the microlens, the transparent medium layer, and the light transmission pinhole are provided so that the imaging pixel has an object-side field of view with a confined angle of view on the object plane in the set area; an image point or an image spot of an object point within the object-side field of view falls within the light transmission pinhole of the light blocking film; and an image point or an image spot of an object point outside the object-side field of view falls outside the light transmission pinhole.

2. The imaging pixel according to claim 1, wherein the microlens is a curved convex lens.

3. The imaging pixel according to claim 1, wherein a line connecting centers of the light transmission pinhole and the microlens is perpendicular to an upper surface of the light blocking film.

4. The imaging pixel according to claim 1, wherein the imaging pixel has an optical axis not perpendicular to an upper surface of the light blocking film.

5. The imaging pixel according to claim 1, wherein the angle of view corresponding to the object-side field of view is less than 10 degrees.

6. The imaging pixel according to claim 1, wherein the light blocking film is a film made of an opaque material, and the light blocking film has a thickness less than 100 um.

7. An image acquisition device, comprising several imaging pixels according to any of claims 1 to 6, which are arranged in an array; and a low-refractive-index layer and a transparent cover plate sequentially disposed on the several imaging pixels from bottom to top, wherein
an image point of an object point within an object-side field of view on an upper surface of the transparent cover plate corresponding to each of the imaging pixels falls within a light transmission pinhole of the light blocking film; and an image point of an object point outside the object-side field of view falls outside the light transmission pinhole.

8. The image acquisition device according to claim 7, wherein between adjacent imaging pixels, a light blocking wall is disposed in a transparent medium layer between a microlens and the light blocking film, to prevent signal crosstalk between the adjacent imaging pixels.

9. The image acquisition device according to claim 7, wherein all of the imaging pixels are located on a same planar substrate, and have parallel optical axes.

10. The image acquisition device according to claim 7, wherein the imaging pixels are located on a curved substrate convex or concave to the transparent cover plate.

11. The image acquisition device according to claim 7, wherein the imaging pixels of the image acquisition device are regularly arranged from inside toward outside with respect to one or some of the imaging pixels as a center, the imaging pixel as the center has an orthogonal object-side field of view, and other imaging pixels each have an oblique object-side field of view; and from the center toward outside, optical axes of the respective imaging pixels gradually oblique outwardly, and angles between the optical axes and an upper surface of the light blocking film gradually decrease.

12. A fingerprint acquisition apparatus, comprising the image acquisition device according to any of claims 7 to 11.

13. A display apparatus supporting a fingerprint acquisition function, comprising several display pixels, wherein the imaging pixel according to any of claims 1 to 6 is disposed between the display pixels.
